(19) Europäisches Patentamt
European Patent Office
Office européen des brevets

(11) EP 4 301 323 B1

(12) EUROPEAN PATENT SPECIFICATION

(45) Date of publication and mention of the grant of the patent:
**29.01.2025 Bulletin 2025/05**

(21) Application number: **22709739.1**

(22) Date of filing: **04.03.2022**

(51) International Patent Classification (IPC):
***A61K 8/19*** *(2006.01)* ***A61K 8/42*** *(2006.01)*
***A61K 8/73*** *(2006.01)* ***A61Q 11/00*** *(2006.01)*
***A61K 8/49*** *(2006.01)*

(52) Cooperative Patent Classification (CPC):
**A61K 8/42; A61K 8/19; A61K 8/494; A61K 8/73; A61Q 11/00;** A61K 2800/43; A61K 2800/58

(86) International application number:
**PCT/EP2022/055497**

(87) International publication number:
**WO 2022/184876 (09.09.2022 Gazette 2022/36)**

(54) **ORAL CARE COMPOSITION**

MUNDPFLEGEZUSAMMENSETZUNG

COMPOSITION DE SOINS BUCCO-DENTAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.03.2021 PCT/CN2021/079381**
**13.04.2021 EP 21168023**

(43) Date of publication of application:
**10.01.2024 Bulletin 2024/02**

(73) Proprietors:
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO SE SI SK SM**
- **Unilever Global IP Limited**
  **Wirral, Merseyside CH62 4ZD (GB)**
  Designated Contracting States:
  **CY DE GB IE IT MT RS TR**

(72) Inventors:
- **JIN, Huajin**
  **6708 WH Wageningen (NL)**
- **LIU, Weining**
  **6708 WH Wageningen (NL)**

(74) Representative: **Tansley, Sally Elizabeth**
**Unilever Patent Group**
**Bronland 14**
**6708 WH Wageningen (NL)**

(56) References cited:
**EP-A1- 3 134 065**
**WO-A1-2019/123171**
**WO-A1-2020/091805**
**WO-A1-2020/234811**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Field of the Invention

The present invention relates to oral care compositions such as toothpastes, powders, gums, mouthwashes and the like. In particular, the present invention relates to oral care compositions comprising benefit agents and a deposition aid for the benefit agents. The invention also relates to the use of such compositions for whitening teeth and/or improving the oral hygiene of an individual.

### Background of the Invention

The enamel layer of the tooth is naturally an opaque white or slightly off-white colour. However, this enamel layer can become stained or discoloured. Many products we consume have a negative impact on our teeth and mouth. Many substances can stain or reduce the whiteness of one's teeth, in particular, certain foods, tobacco products, and fluids such as tea and coffee. These staining and discolouring substances are often able to permeate the enamel layer. This problem occurs gradually over many years, but imparts a noticeable discoloration of the enamel of one's teeth.

Consumers have always had a strong desire for healthy and white teeth. Benefit agents such as optical agents and antimicrobial agents are commonly incorporated in oral care products to provide benefits such as whitening and better oral hygiene. Many oral care products, however, do not provide sufficient benefit agents deposition on teeth. The benefit agents are simply rinsed away during daily oral hygiene routine like tooth brushing and therefore provide little or no benefits.

Therefore, there is continuing need for an oral care composition which delivers improved deposition of benefit agents on teeth to maximize the effectiveness of such benefit agents.

WO 2020/234811 A1 (3M Innovative Properties Company) discloses a composition comprising from 0.2 wt% to 3 wt% total of arginine, glycine, or a combination thereof based on the total weight of the composition; and from 0.3 wt% to 3 wt% total of one or more surfactants, based on the total weight of the composition, of Formula I: $HOCH_2-(CHOH)_n-CH_2NR^1R^2$ (I) wherein $R^1$ and R2 are independently selected from the group consisting of a hydrogen atom, an alkyl group, $C(O)R^3$, and SO2$R^4$; with $R^3$ and $R^4$ being independently selected from the group consisting of an alkyl group, an aryl group, and an aralkyl group; wherein n is an integer from about 2 to about 5.

WO 2020/091805 A1 (Colgate-Palmolive Company) discloses an oral care composition comprising an orally acceptable vehicle and one or more surfactants. The one or more surfactants may include an N-alkyl-N-acylglucamine.

WO 2019/123171 A1 (3M Innovative Properties Company) discloses compositions that include from 0.1wt% to 8wt% of one or more compounds of formula based on the total weight of the composition $HOCH_2-(CHOH)_n-CH_2NR^1R^2$ wherein $R^1$ and R2 are independently selected from the group consisting of a hydrogen atom, an alkyl group, $C(O)R^3$, and $SO_2R^4$; with $R^3$ and $R^4$ being independently selected from the group consisting of an alkyl group, an aryl group, and an aralkyl group; wherein n is an integer from about 2 to about 5; from 5wt% to 95wt% of one or more plant based oils based on the total weight of the composition; from 5wt% to 80wt% water based on the total weight of the composition and from 0.01wt% to 1wt% of one or more nonionic aromatic phenolic preservatives based on the total weight of the composition wherein the composition has a pH from 4.5 to 9.5, the composition is an emulsion, and the composition is edible.

### Summary of the Invention

In a first aspect, the present invention is directed to an oral care composition comprising:

(a) from 0.01 to 10% by weight of a deposition polymer;
(b) a polyhydroxy fatty acid amide surfactant of formula (I):

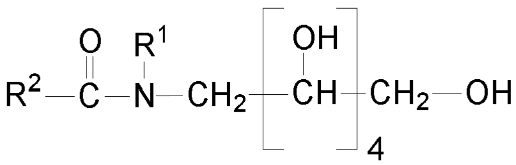

(I)

wherein $R^1$ is a $C_1$-$C_4$ alkyl group or hydroxy-substituted $C_1$-$C_4$ alkyl groups; $R^2$ is a linear or branched, saturated or unsaturated hydrocarbon chain having 15 to 21 carbon atoms; and
(c) a benefit agent selected from optical agents, antimicrobial agents and mixtures thereof;

wherein the deposition polymer comprises xanthan gum, sodium alginate, pectin, modified starch, gelatin, carrageenan, gellan gum, fenugreek gum, konjac gum, tragacanth gum, karaga gum, γ-polyglutamic acid, heparin sodium salt, a copolymer of methyl vinyl ether and maleic anhydride, polyacrylic acid, polystyrene sulfonate, deoxyribonucleic acids, poly(allylamine), locust bean gum, guar gum, polyglycolic acid, polylactic acid, poly(glycolic-co-lactic acid), chitosan, humic acid, phytate acid, derivatives or mixtures thereof.

In a second aspect, the present invention is directed to a packaged oral care product comprising the oral care composition of the first aspect of this invention.

In a third aspect, the present invention is directed to a method of whitening teeth and/or improving the oral hygiene of an individual comprising the steps of applying the composition of any embodiment of the first aspect to at least one surface of the teeth of the individual.

All other aspects of the present invention will more readily become apparent upon considering the detailed description and examples which follow.

## Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

The oral care composition of the invention comprises a deposition polymer. The term "deposition polymer", as used herein, means a polymer which aids deposition of benefit agents from the continuous phase of the composition onto the surface of teeth during use of the composition. Use of the composition in the context of this invention typically involves application of the composition to the oral cavity, followed by brushing and/or rinsing. The deposition polymer enhances the deposition of the benefit agents onto the surfaces of teeth and thereby enhances the performance of the composition. Suitable deposition polymers for use in the invention may be naturally or synthetically-derived.

The deposition polymers for use in the invention include, xanthan gum, sodium alginate, pectin, modified starch, gelatin, carrageenan, gellan gum, fenugreek gum, konjac gum, tragacanth gum, karaga gum, γ-polyglutamic acid, heparin sodium salt, a copolymer of methyl vinyl ether and maleic anhydride, polyacrylic acid, polystyrene sulfonate, deoxyribonucleic acids, poly(allylamine), locust bean gum, guar gum, polyglycolic acid, polylactic acid, poly(glycolic-co-lactic acid), chitosan, humic acid, phytate acid, derivatives or mixtures thereof. Preferably the deposition polymer comprises xanthan gum, sodium alginate, pectin, modified starch, gelatin, carrageenan, gellan gum, fenugreek gum, konjac gum, tragacanth gum, karaga gum, γ-polyglutamic acid, phytate acid, derivatives or mixtures thereof.

It is preferred that the deposition polymer is xanthan gum and/or its derivatives.

Xanthan gum is an extracellular polysaccharide secreted by the microorganism *Xanthomonas campestris.* It is a linear (1, 4)-linked β-D-glucose backbone (as in cellulose) with a trisaccharide side chain on every other glucose at C-3. This side chain comprises a glucuronic acid residue linked (1, 4) to a terminal mannose unit and (1, 2) to a second mannose that connects to the backbone. Approximately 50% of the terminal mannose residues are pyruvated and the non-terminal residue usually carries an acetyl group at C-6.

The structure of xanthan gum is given below:

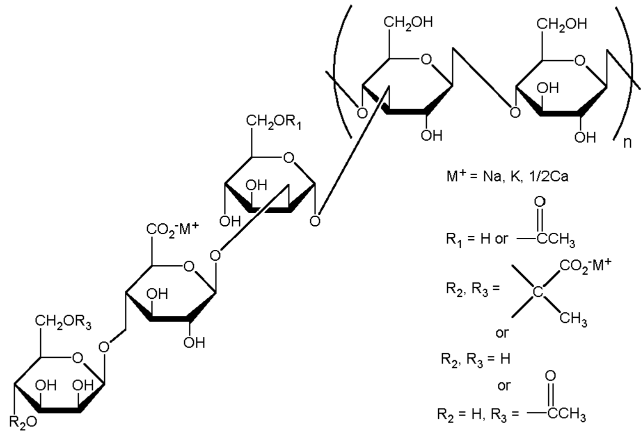

Wherein n ranges from 10 to 10,000, preferably from 100 to 8,000, more preferably from 500 to 5,000, most preferably from 1,000 to 3,000.

The xanthan gum derivatives can be prepared by etherification, esterification, acetalation, amidation or oxidation of non-derivatized xanthan gum. Preferred xanthan gum derivatives are prepared by reacting non-derivatized gum with quaternary ammonium compounds in an amount equal to or greater than the stoichiometric amount required for complete derivatization. The quaternary ammonium compounds are preferably having a single alkyl or alkenyl substituent containing from 13 to 24 carbon atoms, and/or two alkyl or alkenyl substituents of 12 to 24 carbon atoms per substituent. Most preferably, the quaternary ammonium compound is alkyl dimethylbenzylammonium chloride with an alkyl group containing from 13 to 24 carbon atoms.

The oral care composition comprises the deposition polymer in an amount of from 0.01 to 10%, preferably from 0.02 to 5%, more preferably from 0.05 to 3% and most preferably from 0.05 to 2%, based on total weight of the oral care composition and including all ranges subsumed therein.

The polyhydroxy fatty acid amide surfactant suitable for use the invention is a non-ionic surfactant represented by the general formula (I):

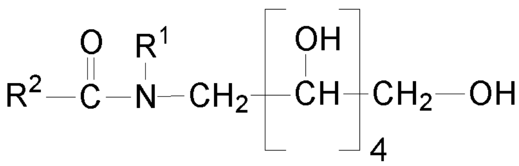

(I)

wherein $R^1$ is a $C_1$-$C_4$ alkyl group or hydroxy-substituted $C_1$-$C_4$ alkyl groups; $R^2$ is a linear or branched, saturated or unsaturated hydrocarbon chain having 15 to 21 carbon atoms.

$R^1$ is preferably a $C_1$-$C_2$ alkyl group, more preferably is a methyl group.

$R^2$ is preferably an alkyl group, an alkenyl group, an aryl group, or an aralkyl group having 15 to 21 carbon atoms, more preferably 15 to 19 carbon atoms and most preferably 16 to 19 carbon atoms.

It is preferred that $R^1$ is a methyl group and $R^2$ is an alkyl group having 15 to 21 carbon atoms, more preferably 15 to 19 carbon atoms, and most preferably 16 to 19 carbon atoms.

Preferably, the polyhydroxy fatty acid amide surfactant comprises at least 60% by weight of a polyhydroxy fatty acid amide with $R^1$ being a methyl group and $R^2$ being a $C_{17}$ alkyl group, more preferably from 70 to 100%, more preferably still from 80 to 100% and most preferably from 90 to 100%, based on total weight of the polyhydroxy fatty acid amide.

It is particularly preferred that the surfactant is polyhydroxy fatty acid amide with $R^1$ being a methyl group and $R^2$ being a $C_{17}$ alkyl group.

The composition of the present invention may additionally have small fractions of polyhydroxy fatty acid amide having short hydrocarbon chains, in particular wherein $R^2$ is a $C_1$-$C_4$, $C_5$, $C_7$, $C_9$, $C_{11}$, $C_{13}$ alkyl group or an $C_2$, $C_3$, $C_5$, $C_7$, $C_9$, $C_{11}$, $C_{13}$ alkenyl group.

One example of a polyhydroxy fatty acid amide surfactant suitable for use in this invention is sunfloweroyl methyl glucamide commercially available as GlucoPure® Sense from Clariant International Ltd.

The oral care composition typically comprises the polyhydroxy fatty acid amide in an amount of from 0.01 to 10% by weight of the composition, more preferably from 0.1 to 8% and most preferably from 0.5 to 5%, based on total weight of the oral care composition and including all ranges subsumed therein.

Benefit agent, as used herein, means an active typically delivered to human teeth and/or the oral cavity including

the gums to enhance or improve a characteristic of those dental tissues. The only limitation with respect to the benefit agent that may be used in this invention is that the same is suitable for use in the mouth.

Typically benefit agent includes, for example, optical agents, biomineralization agents, adsorbing agents, antimicrobial agents, gum health agents, desensitizing agents, anti-calculus agents, freshness agents or mixtures thereof. Preferably the benefit agent is selected from optical agents, antimicrobial agents and mixtures thereof.

Optical agents may be selected from one or more of whitening agents and pearlescent agents. When the optical agent is a whitening agent, it is preferable that the whitening agent is a dye or a pigment or a combination of the two. The pigment according to the invention is a shade/material which is insoluble in the relevant medium, at the relevant temperature. This is in contrast to dyes which are soluble. The term "relevant medium", as used herein, refers to human saliva, the liquid medium in which the composition is used, at the temperature of the oral cavity during brushing of the teeth, i.e. up to 37°C. The relevant medium may also be water and the relevant temperature to be 25°C. Preferred pigment or dye for use in the present invention is violet or blue having a hue angle, h, in the CIELAB system of from 220 to 320 degrees, preferably from 250 to 290 degrees. When the whitening agent is a pigment, it is preferable that the pigment is violet or blue selected from one or more of those listed in the Colour Index International as pigment blue 1 through to pigment blue 83 and pigment violet 1 through to pigment violet 56. Other suitable pigments are pigment ultramarine blue and ultramarine violet. While the preferred pigment is blue or violet, the same effect may be achieved through mixing pigments outside of this hue angle range. For example, such a hue angle may also be obtained by mixing a red and green-blue pigment to yield a blue or violet shaded pigment. It is particularly preferred that the pigment is a blue pigment. A preferred class of blue pigments suitable for use in the invention are organic blue pigments such as phthalocyanine blue pigments. It is especially preferred that the pigment is phthalocyanine blue pigment selected from alpha copper phthalocyanines Pigment Blue 15, Pigment Blue 15:1, Pigment Blue 15:2, Pigment Blue 15:3, Pigment Blue 15:4, Pigment Blue 15:6 and mixtures thereof, more preferably Pigment Blue 15, Pigment Blue 15:1, Pigment Blue 15:2 and mixtures thereof. Most preferably the pigment is Pigment Blue 15:1. A commercially available example is Cosmenyl Blue A4R from Clariant. When the whitening agent is a dye, it is preferable that the dye is a blue dye, more preferably FD&C Blue 1, Patent Blue V or Gardenia Blue.

In another preferred embodiment, the whitening agent is a particulate whitening agent having a refractive index of from 1.9 to 4.0. The refractive index is quoted at a temperature of 25°C and a wavelength of 589 nm. Suitable materials to provide such a high refractive index are metal compounds and preferred are compounds where the metal is selected from zinc (Zn), titanium (Ti), zirconium (Zr) or a mixture thereof. Preferably, the metal compound is (or at least comprises) a metal oxide such as titanium dioxide ($TiO_2$), zinc oxide (ZnO), zirconium dioxide ($ZrO_2$) or a mixture thereof. Preferably, the particulate whitening agent is a composite particle. The term "Composite particle", as used herein, means a particle comprising a first component core and a second component coating wherein the core and coating are composed of different materials. The composite particle comprises a first component core and a second component coating. Particularly suitable core materials are metal compounds and preferred are compounds where the metal is selected from zinc (Zn), titanium (Ti), zirconium (Zr) or a mixture thereof. Preferably, the metal compound is (or at least comprises) a metal oxide such as titanium dioxide ($TiO_2$), zinc oxide (ZnO), zirconium dioxide ($ZrO_2$) or a mixture thereof. The second component coating comprises material suitable to adhere to tooth enamel, dentin or both. Typically the coating material comprises the element calcium, and optionally, other metals like potassium, sodium, aluminium, magnesium as well as mixtures thereof whereby such optional metals are provided as, for example, sulphates, lactates, oxides, carbonates or silicates. Optionally, the coating material may be aluminium oxide or silica. In a preferred embodiment, the second component coating can comprise, for example, calcium phosphate, calcium gluconate, calcium oxide, calcium lactate, calcium carbonate, calcium hydroxide, calcium sulphate, calcium carboxymethyl cellulose, calcium alginate, calcium salts of citric acid, calcium silicate, mixture thereof or the like. Calcium silicate is particularly preferred. In an especially preferred embodiment, the particulate whitening agent is titanium dioxide coated with calcium silicate.

In another preferred embodiment, the optical agent is a pearlescent agent. The term "pearlescent", as used herein, means an agent in the form of particles which reflects and partially transmits the incident light. Suitable pearlescent agent for use in the present invention includes mica, bismuth oxychloride or mixtures thereof. Preferably the pearlescent agent is formed by coating one or more metal oxide layers onto natural or synthetic mica flakes. Examples of suitable mica include, for example, muscovite, phlogopite, fluorophlogopite, biotite or mixtures thereof. Examples of suitable metal oxides include, for example, $TiO_2$, $Fe_2O_3$, $TiFe_2O_5$, $Fe_3O_4$, $Cr_2O_3$, $Al_2O_3$, $SiO_2$, $ZrO_2$, ZnO, $SnO_2$, CoO, $Co_3O_4$, $VO_2$, $V_2O_3$, $Sn(Sb)O_2$, titanium suboxides or mixtures thereof. One example of a preferred pearlescent agent is mica coated with $TiO_2$.

Antimicrobial agents may be selected from one or more of metal compounds where the metal is selected from zinc, copper, silver or a mixture thereof, triclosan, triclosan monophosphate, triclocarban, curcumin, quaternary ammonium compounds, bisbiguanides and long chain tertiary amines, preferably zinc compounds including zinc oxide, zinc chloride, zinc acetate, zinc ascorbate, zinc sulphate, zinc nitrate, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate or a mixture thereof. Zinc citrate is particularly preferred.

It is preferred that the benefit agent is an optical agent selected from pigments, dyes, mica, mica coated with $TiO_2$,

titanium dioxide, titanium dioxide coated with calcium silicate and mixtures thereof. More preferably the benefit agent is a phthalocyanine blue pigment is selected from alpha copper phthalocyanines Pigment Blue 15, Pigment Blue 15:1, Pigment Blue 15:2 and mixtures thereof. Pigment Blue 15:1 is particularly preferred.

It is also preferred that the benefit agent is zinc compounds selected from zinc oxide, zinc chloride, zinc acetate, zinc ascorbate, zinc sulphate, zinc nitrate, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate and mixtures thereof. Zinc citrate is particularly preferred.

The oral care composition of the present invention may comprise a single benefit agent or a mixture of two or more benefit agents. Typically, the benefit agent is present in an amount from 0.01 to 60%, and more preferably, from 0.05 to 40%, and most preferably **from 0.1 to** 30% by total weight of the oral care composition and including all ranges subsumed therein.

When the benefit agent is a pigment, the pigment is preferably present at a level of from 0.001 to 1% by weight of the oral care composition, more preferably from 0.01 to 0.5%, most preferably from 0.02 to 0.2%, based on total weight of the oral care composition and including all ranges subsumed therein.

Preferably, the oral care composition has a pH from 4.0 to 10.0, more preferably from 5.0 to 9.0, and most preferably from 5.5 to 8.0. The pH of oral care composition is measured when 5 parts by weight of the composition is uniformly dispersed and/or dissolved in 20 parts by weight pure water at 25°C. In particular, the pH may be measured by manually mixing 5 g oral care composition with 20 mL water for 30 s, then immediately testing the pH with indicator or a pH meter.

The oral care composition may comprise other surfactants in addition to the polyhydroxy fatty acid amide. Suitable surfactants include anionic surfactants, such as the sodium, magnesium, ammonium or ethanolamine salts of $C_8$ to $C_{18}$ alkyl sulphates (for example sodium lauryl sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides. Other suitable surfactants include non-ionic surfactants, such as polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is an anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine, sodium methyl cocoyl taurate or mixtures thereof.

When the oral care composition comprises surfactants in addition to the polyhydroxy fatty acid amide, the surfactant is typically present at a level from 0.01 to 7% by weight of the composition, more preferably from 0.1 to 7% and most preferably from 0.5 to 5%, based on total weight of the oral care composition and including all ranges subsumed therein.

The oral care composition of the present invention may comprise a physiologically acceptable carrier. Water is the most common carrier for this invention. The carrier may further comprise at least thickener, humectant or a combination thereof.

Thickener may be used in this invention in addition to the deposition polymer which is included in the oral care composition and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, silica based thickeners including silica aerogels, magnesium aluminum silicate (e.g., Veegum) and mixtures thereof.

Sodium carboxymethyl cellulose is typically preferred. The copolymer which is included in the composition can also behave as a thickener.

In an especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickener typically makes up from 0.01 to about 10%, more preferably from 0.1 to 9%, and most preferably, from 0.1 to 5% by weight of the oral care composition, based on total weight of the composition and including all ranges subsumed therein.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The carrier is typically present at a level of from 10 to 90% by weight of the oral care composition, more preferably 25 to 80%, and most preferably from 30 to 70% by weight of the composition, based on total weight of the composition and

including all ranges subsumed therein.

The oral care composition of the present invention may contain a variety of other ingredients in addition to the benefit agent that is included in the composition, which are common in the art to enhance physical properties and performance. These ingredients include preservatives, pH-adjusting agents, sweetening agents, particulate abrasive materials, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of benefiting teeth of an individual comprising the steps of applying the composition to at least one surface of the teeth of an individual. In particular, the oral care composition of this invention can be used in a method of whitening teeth and/or improving oral hygiene of an individual comprising the steps of applying the composition to at least one surface of the teeth of the individual. Preferably, the method is non-therapeutic. The oral care composition of this invention may additionally or alternatively be used for whitening teeth and/or improving oral hygiene of an individual and/or used in the manufacture of a medicament for whitening teeth and/or improving oral hygiene of an individual. Preferably, the use is non-therapeutic.

Typically, the composition will be packaged. In toothpaste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The oral care composition of the invention is preferably a toothpastes or gel. When the oral care composition is a toothpaste or gel, the same typically has a viscosity from about 30,000 to 180,000 centipoise, and preferably, from 60,000 to 170,000 centipoise, and most preferably, from 65,000 to 165,000 centipoise, taken at room temperature (25°C) with a Brookfield Viscometer, Spindle No.93/94 and at a speed of 5 rpm for 1 minute.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day.

The following examples are provided to facilitate an understanding of the present invention. The examples are not provided to limit the scope of the claims.

## Examples

### Example 1

This example demonstrated the deposition of pigments. All ingredients are expressed by weight percent of the total formulation, and as level of active ingredient.

**Table 1**

| Ingredient | Samples | | | | | | | |
|---|---|---|---|---|---|---|---|---|
| | 1 | 2 | 3 | 4 | 5 | 6 | 7 | 8 |
| Water | To 100 | To 100 | To 100 | To 100 | To 100 | To 100 | To 100 | To 100 |
| Blue Covarine[a] | 0.025 | 0.025 | 0.025 | 0.025 | 0.025 | 0.025 | 0.025 | 0.025 |
| Xanthan gum | 0.1 | 0.1 | 0.1 | 0.1 | 0.1 | -- | -- | -- |
| Sodium lauryl sulfate | 1.8 | -- | -- | -- | -- | -- | -- | -- |
| Capryloyl/caproyl methyl glucamide[b] | -- | 1.8 | -- | -- | -- | 1.8 | -- | -- |
| Lauroyl/myristoyl methyl glucamide[c] | -- | -- | 1.8 | -- | -- | -- | 1.8 | -- |
| Sunfloweroyl methyl glucamide[d] | -- | -- | -- | 1.8 | -- | -- | -- | 1.8 |

a. Dispersion of C.I. 74160 (pigment blue 15:1) in water/glycerol under the trade name Cosmenyl Blue A4R from Clariant.
b. Commercially available capryloyl/caproyl methyl glucamide under the trade name Glucotain® Clear from Clariant
c. Commercially available lauroyl/myristoyl methyl glucamide under the trade name Glucotain® Flex from Clariant.
d. Commercially available sunfloweroyl methylglucamide under the trade name Glucopure® Sense from Clariant.

*Methods*

To evaluate the deposition of pigments, the following in vitro test was carried out. The colour difference expressed as the CIELAB delta E (ΔE) value was calculated based on L*, a*, b* values measured using DigiEye (VeriVide, England) with the following formula:

$$\Delta E = \sqrt{\Delta L^2 + \Delta a^2 + \Delta b^2}$$

Hydroxyapatite (HAP) discs were used as substrates. The baseline L*, a*, b* of the HAP discs was measured by DigiEye (VeriVide, England). Four HAP discs were used for each sample. The HAP discs were soaked in de-ionized (DI) water for 1 hour before they were immersed in different samples for 24 hours. Thereafter the HAP discs were rinsed with 40 mL DI water and shaken by hand gently for 6 times. The residual water on HAP discs was absorbed with tissue. After this step the L*, a*, b* values of each HAP disc were remeasured based on which the delta E (ΔE) from baseline were calculated and statistically analyzed.

*Results*

The results are summarized in table 2. The higher the average ΔE value the more deposition of the pigment.

**Table 2**

| | Samples | | | | | | | |
|---|---|---|---|---|---|---|---|---|
| | 1 | 2 | 3 | 4 | 5 | 6 | 7 | 8 |
| AverageΔE | 30.41 | 17.83 | 30.95 | 63.24 | 26.80 | 20.68 | 9.65 | 24.64 |

It can be seen from the results that sample 4 showed significantly higher ΔE value ($p<0.05$) compared to other samples, indicating much better deposition of the pigment.

## Claims

**1.** An oral care composition comprising:

(a) from 0.01 to 10% by weight of a deposition polymer;
(b) a polyhydroxy fatty acid amide surfactant of formula (I):

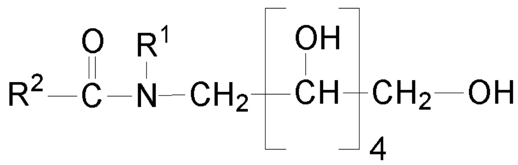

(I)

wherein $R^1$ is a $C_1$-$C_4$ alkyl group or hydroxy-substituted $C_1$-$C_4$ alkyl groups;
$R^2$ is a linear or branched, saturated or unsaturated hydrocarbon chain having 15 to 21 carbon atoms; and

(c) a benefit agent selected from optical agents, antimicrobial agents and mixtures thereof;

wherein the deposition polymer comprises xanthan gum, sodium alginate, pectin, modified starch, gelatin, carrageenan, gellan gum, fenugreek gum, konjac gum, tragacanth gum, karaga gum, γ-polyglutamic acid, heparin sodium salt, a copolymer of methyl vinyl ether and maleic anhydride, polyacrylic acid, polystyrene sulfonate, deoxyribonucleic acids, poly(allylamine), locust bean gum, guar gum, polyglycolic acid, polylactic acid, poly(glycolic-co-lactic acid), chitosan, humic acid, phytate acid, derivatives or mixtures thereof.

**2.** The oral care composition according to claim 1, wherein the deposition polymer comprises xanthan gum, sodium alginate, pectin, modified starch, gelatin, carrageenan, gellan gum, fenugreek gum, konjac gum, tragacanth gum, karaga gum, γ-polyglutamic acid, phytate acid, derivatives or mixtures thereof, preferably xanthan gum and/or its

derivatives.

3. The oral care composition according to claim 1 or claim 2, wherein the composition comprises the deposition polymer in an amount of from 0.02 to 5% by weight of the composition, preferably from 0.05 to 3%.

4. The oral care composition according to any of the preceding claims, wherein $R^2$ is an alkyl group, an alkenyl group, an aryl group, or an aralkyl group having 15 to 21 carbon atoms, preferably 15 to 19 carbon atoms.

5. The oral care composition according to any of the preceding claims, wherein $R^1$ is a $C_1$-$C_2$ alkyl group, preferably a methyl group.

6. The oral care composition according to any of the preceding claims, wherein $R^1$ is a methyl group, $R^2$ is an alkyl group having 15 to 21 carbon atoms, preferably 15 to 19 carbon atoms.

7. The oral care composition according to any of the preceding claims, wherein the polyhydroxy fatty acid amide surfactant comprises at least 60% by weight of a polyhydroxy fatty acid amide with $R^1$ being a methyl group and $R^2$ being a $C_{17}$ alkyl group, preferably from 70 to 100%, more preferably from 80 to 100%

8. The oral care composition according to any of the preceding claims, wherein the polyhydroxy fatty acid amide surfactant is a polyhydroxy fatty acid amide with $R^1$ being a methyl group and $R^2$ being a $C_{17}$ alkyl group.

9. The oral care composition according to any of the preceding claims, wherein the composition comprises the polyhydroxy fatty acid amide surfactant in an amount of from 0.01 to 10% by weight of the composition, preferably from 0.1 to 8%.

10. The oral care composition according to any of the preceding claims, wherein the benefit agent is an optical agent selected from pigments, dyes, mica, mica coated with $TiO_2$, titanium dioxide, titanium dioxide coated with calcium silicate and mixtures thereof.

11. The oral care composition according to claim 10, wherein the benefit agent is a pigment having a hue angle, h, in the CIELAB system of from 220 to 320 degrees, preferably from 250 to 290 degrees.

12. The oral care composition according to claim 11, wherein the pigment is a blue pigment, preferably a phthalocyanine blue pigment.

13. The oral care composition according to claim 12, wherein the phthalocyanine blue pigment is selected from alpha copper phthalocyanines Pigment Blue 15, Pigment Blue 15:1, Pigment Blue 15:2 and mixtures thereof, preferably Pigment Blue 15:1.

14. The oral care composition according to any of the preceding claims, wherein benefit agent is an antimicrobial agent, preferably zinc compounds selected from zinc oxide, zinc chloride, zinc acetate, zinc ascorbate, zinc sulphate, zinc nitrate, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate and mixtures thereof, more preferably zinc citrate.

15. A method of whitening teeth and/or improving the oral hygiene of an individual comprising the steps of applying the composition according to any of the preceding claims to at least one surface of the teeth of the individual.

## Patentansprüche

1. Mundpflegezusammensetzung, umfassend:

   (a) 0,01 bis 10 Gewichts-% eines Abscheidungspolymers;
   (b) ein Polyhydroxyfettsäureamid-Tensid der Formel (I):

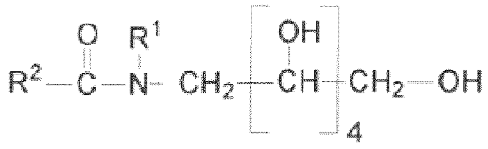

(I)

worin darstellen:

$R^1$ eine $C_1$-$C_4$-Alkylgruppe oder Hydroxy-substituierte $C_1$-$C_4$-Alkylgruppen;
$R^2$ eine linear oder verzweigte, gesättigte oder ungesättigte Kohlenwasserstoffkette mit 15 bis 21 Kohlenstoffatomen; und

(c) ein Vorteilsmittel, ausgewählt unter optischen Mitteln, antimikrobiellen Mitteln und Mischungen davon;

wobei das Abscheidungspolymer umfasst Xanthangummi, Natriumalginat, Pektin, modifizierte Stärke, Gelatine, Carrageen, Gellangummi, Bockshornkleegummi, Konjakgummi, Traganthgummi, Karagagummi, γ-Polyglutaminsäure, Heparin-Natriumsalz, ein Copolymer aus Methylvinylether und Maleinsäureanhydrid, Polyacrylsäure, Polystyrolsulfonat, Desoxyribonukleinsäuren, Poly(allylamin), Johannesbrotkernmehl, Guarkernmehl, Polyglykolsäure, Polymilchsäure, Poly(glykol-co-Milchsäure), Chitosan, Huminsäure, Phytatsäure, Derivate oder Mischungen davon.

**2.** Mundpflegezusammensetzung nach Anspruch 1, wobei das Abscheidungspolymer Xanthangummi, Natriumalginat, Pektin, modifizierte Stärke, Gelatine, Carrageen, Gellangummi, Bockshornkleegummi, Konjakgummi, Traganthgummi, Karagagummi, γ-Polyglutaminsäure, Phytatsäure, Derivate oder Mischungen davon, bevorzugt Xanthangummi und/oder Derivate davon umfasst.

**3.** Mundpflegezusammensetzung nach Anspruch 1 oder Anspruch 2, wobei die Zusammensetzung das Abscheidungspolymer in einer Menge von 0,02 bis 5 Gewichts-% der Zusammensetzung, bevorzugt von 0,05 bis 3%, umfasst.

**4.** Mundpflegezusammensetzung nach einem der vorhergehenden Ansprüche, wobei $R^2$ eine Alkylgruppe, eine Alkenylgruppe, eine Arylgruppe oder eine Aralkylgruppe mit 15 bis 21 Kohlenstoffatomen, bevorzugt mit 15 bis 19 Kohlenstoffatomen, ist.

**5.** Mundpflegezusammensetzung nach einem der vorhergehenden Ansprüche, wobei $R^1$ eine $C_1$-$C_2$-Alkylgruppe, bevorzugt eine Methylgruppe, ist.

**6.** Mundpflegezusammensetzung nach einem der vorhergehenden Ansprüche, wobei $R^1$ eine Methylgruppe, $R^2$ eine Alkylgruppe mit 15 bis 21 Kohlenstoffatomen, bevorzugt mit 15 bis 19 Kohlenstoffatomen, ist.

**7.** Mundpflegezusammensetzung nach einem der vorhergehenden Ansprüche, wobei das Polyhydroxyfettsäureamid-Tensid mindestens 60 Gewichts-% eines Polyhydroxyfettsäureamids mit $R^1$ in Form einer Methylgruppe und $R^2$ in Form einer $C_{17}$-Alkylgruppe, bevorzugt 70 bis 100%, bevorzugter 80 bis 100%, umfasst.

**8.** Mundpflegezusammensetzung nach einem der vorhergehenden Ansprüche, wobei das Polyhydroxyfettsäureamid-Tensid ein Polyhydroxyfettsäureamid mit $R^1$ in Form einer Methylgruppe und $R^2$ in Form einer $C_{17}$-Alkylgruppe ist.

**9.** Mundpflegezusammensetzung nach einem der vorhergehenden Ansprüche, wobei die Zusammensetzung das Polyhydroxyfettsäureamid-Tensid in einer Menge von 0,01 bis 10 Gewichts-% der Zusammensetzung, bevorzugt von 0,1 bis 8%, umfasst.

**10.** Mundpflegezusammensetzung nach einem der vorhergehenden Ansprüche, wobei das Vorteilsmittel ein optisches Mittel ist, das unter Pigmenten, Farbstoffen, Glimmer, $TiO_2$-beschichtetem Glimmer, Titandioxid, Calciumsilikatbeschichtetem Titandioxid und Mischungen davon ausgewählt ist.

**11.** Mundpflegezusammensetzung nach Anspruch 10, wobei das Vorteilsmittel ein Pigment mit einem Farbtonwinkel h im CIELAB-System von 220 bis 320 Grad, bevorzugt von 250 bis 290 Grad, ist.

**12.** Mundpflegezusammensetzung nach Anspruch 11, wobei das Pigment ein Blaupigment, bevorzugt ein Phthalocyanin-Blaupigment, ist.

**13.** Mundpflegezusammensetzung nach Anspruch 12, wobei das Phthalocyanin-Blaupigment unter den alpha-Kupferphthalocyaninen Pigment Blue 15, Pigment Blue 15:1, Pigment Blue 15:2 und Mischungen davon, bevorzugt Pigment Blue 15:1, ausgewählt ist.

**14.** Mundpflegezusammensetzung nach einem der vorhergehenden Ansprüche, wobei das Vorteilsmittel ein antimikrobielles Mittel ist, bevorzugt Zink-Verbindungen, ausgewählt unter Zinkoxid, Zinkchlorid, Zinkacetat, Zinkascorbat, Zinksulfat, Zinknitrat, Zinkcitrat, Zinklaktat, Zinkperoxid, Zinkfluorid, Zinkammoniumsulfat, Zinkbromid, Zinkiodid, Zinkgluconat, Zinktartrat, Zinksuccinat, Zinkformiat, Zinkphenolsulfonat, Zinksalicylat, Zinkglycerophosphat und Mischungen davon, bevorzugter Zinkcitrat.

**15.** Verfahren zum Aufhellen der Zähne und/oder zum Verbessern der Mundhygiene einer Person, umfassend die Schritte des Auftragens der Zusammensetzung nach einem der vorhergehenden Ansprüche auf mindestens eine Oberfläche der Zähne der Person.

## Revendications

**1.** Composition d'hygiène buccale comprenant :

(a) 0,01 à 10 % en poids d'un polymère de déposition ;
(b) un tensioactif amide d'acide gras polyhydroxylé de formule (I) :

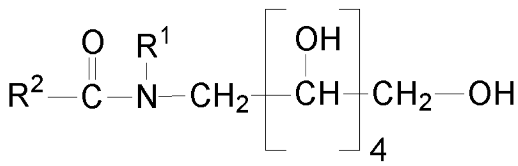

(I)

dans laquelle $R^1$ est un groupe alkyle en $C_1$ à $C_4$ ou un groupe alkyle en $C_1$ à $C_4$ à substitution hydroxy ; $R^2$ est une chaîne hydrocarbonée ramifiée, saturée ou insaturée ayant 15 à 21 atomes de carbone ; et

(c) un agent bénéfique choisi parmi les agents optiques, les agents antimicrobiens et leurs mélanges ;

dans laquelle le polymère de déposition comprend la gomme xanthane, l'alginate de sodium, la pectine, l'amidon modifié, la gélatine, la carraghénane, la gomme gellane, la gomme de fenugrec, la gomme konjac, la gomme adragante, la gomme karaya, un acide poly(γ-glutamique), le sel sodique d'héparine, un copolymère de méthylvinyléther et d'anhydride maléique, un poly(acide acrylique), un polystyrènesulfonate, les acides désoxyribonucléiques, une polyallylamine, la gomme de caroube, la gomme de guar, un poly(acide glycolique), un poly(acide lactique), un poly(acide glycolique-co-lactique), le chitosane, l'acide humique, un phytate acide, ou leurs dérivés ou mélanges.

**2.** Composition d'hygiène buccale selon la revendication 1, dans laquelle le polymère de déposition comprend la gomme xanthane, l'alginate de sodium, la pectine, l'amidon modifié, la gélatine, la carraghénane, la gomme gellane, la gomme de fenugrec, la gomme konjac, la gomme adragante, la gomme karaya, un acide poly(γ-glutamique), un phytate acide, ou leurs dérivés ou mélanges, de préférence la gomme xanthane et/ou ses dérivés.

**3.** Composition d'hygiène buccale selon la revendication 1 ou la revendication 2, laquelle composition comprend le polymère de déposition en une quantité de 0,02 à 5 %, de préférence de 0,05 à 3 %, en poids de la composition.

**4.** Composition d'hygiène buccale selon l'une quelconque des revendications précédentes, dans laquelle $R^2$ est un groupe alkyle, un groupe alcényle, un groupe aryle, ou un groupe aralkyle ayant 15 à 21 atomes de carbone, de préférence 15 à 19 atomes de carbone.

**5.** Composition d'hygiène buccale selon l'une quelconque des revendications précédentes, dans laquelle $R^1$ est un groupe alkyle en $C_1$ à $C_2$, de préférence un groupe méthyle.

**6.** Composition d'hygiène buccale selon l'une quelconque des revendications précédentes, dans laquelle $R^1$ est un groupe méthyle, $R^2$ est un groupe alkyle ayant 15 à 21 atomes de carbone, de préférence 15 à 19 atomes de carbone.

7. Composition d'hygiène buccale selon l'une quelconque des revendications précédentes, dans laquelle le tensioactif amide d'acide gras polyhydroxylé comprend au moins 60 %, de préférence 70 à 100 %, mieux encore 80 à 100 % en poids, d'un amide d'acide gras polyhydroxylé où $R^1$ est un groupe méthyle et $R^2$ est un groupe alkyle en $C_{17}$.

8. Composition d'hygiène buccale selon l'une quelconque des revendications précédentes, dans laquelle le tensioactif amide d'acide gras polyhydroxylé est un amide d'acide gras polyhydroxylé où $R^1$ est un groupe méthyle et $R^2$est un groupe alkyle en $C_{17}$.

9. Composition d'hygiène buccale selon l'une quelconque des revendications précédentes, laquelle composition comprend le tensioactif amide d'acide gras polyhydroxylé en une quantité de 0,01 à 10 %, de préférence de 0,1 à 8 %, en poids de la composition.

10. Composition d'hygiène buccale selon l'une quelconque des revendications précédentes, dans laquelle l'agent bénéfique est un agent optique choisi parmi les pigments, les colorants, le mica, le mica revêtu de $TiO_2$, le dioxyde de titane, le dioxyde de titane revêtu de silicate de calcium, et leurs mélanges.

11. Composition d'hygiène buccale selon la revendication 10, dans laquelle l'agent bénéfique est un pigment ayant un angle de teinte, h, dans le système CIELAB, de 220 à 320 degrés, de préférence de 250 à 290 degrés.

12. Composition d'hygiène buccale selon la revendication 11, dans laquelle le pigment est un pigment bleu, de préférence un pigment bleu de phtalocyanine.

13. Composition d'hygiène buccale selon la revendication 12, dans laquelle le pigment bleu de phtalocyanine est choisi parmi les phtalocyanines de cuivre alpha Pigment Blue 15, Pigment Blue 15:1, Pigment Blue 15:2 et leurs mélanges, de préférence le Pigment Blue 15:1.

14. Composition d'hygiène buccale selon l'une quelconque des revendications précédentes, dans laquelle l'agent bénéfique est un agent antimicrobien, de préférence des composés du zinc choisis parmi l'oxyde de zinc, le chlorure de zinc, l'acétate de zinc, l'ascorbate de zinc, le sulfate de zinc, le nitrate de zinc, le citrate de zinc, le lactate de zinc, le peroxyde de zinc, le fluorure de zinc, l'ammoniosulfate de zinc, le bromure de zinc, l'iodure de zinc, le gluconate de zinc, le tartrate de zinc, le succinate de zinc, le formiate de zinc, le phénolsulfonate de zinc, le salicylate de zinc, le glycérophosphate de zinc et leurs mélanges, mieux encore le citrate de zinc.

15. Méthode de blanchiment des dents et/ou d'amélioration de l'hygiène buccale d'un individu, comprenant l'étape d'application de la composition selon l'une quelconque des revendications précédentes à au moins une surface des dents de l'individu.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020234811 A1
- WO 2020091805 A1
- WO 2019123171 A1


### Non-patent literature cited in the description


- *CHEMICAL ABSTRACTS*, 9004-32-4